# EUROPEAN PATENT SPECIFICATION

(11) **EP 2 115 116 B1**
(45) Date of publication and mention of the grant of the patent: **24.01.2018**
(21) Application number: 08731431.6
(22) Date of filing: 05.03.2008
(51) Int. Cl.: C12M 1/12, C12M 3/06

(54) **METHODS TO CONTROL CELL MOVEMENT IN HOLLOW FIBER BIOREACTORS**
VERFAHREN ZUR KONTROLLE DER ZELLBEWEGUNG IN HOHLFASERBIOREAKTOREN
PROCÉDÉ DE COMMANDE DE DÉPLACEMENT DE CELLULES DANS DES BIORÉACTEURS À FIBRES CREUSES

(30) Priority: 05.03.2007 US 892903 P; 05.03.2007 US 892962 P; 05.03.2007 US 892981 P; 12.04.2007 US 911393 P; 11.09.2007 US 971511 P; 11.09.2007 US 971494 P
(43) Date of publication of application: 11.11.2009
(73) Proprietor: Terumo BCT, Inc., Lakewood, CO 80215 (US)
(72) Inventor: ANTWILER, Glen, Delbert, Lakewood, CO 80215 (US)
(74) Representative: J A Kemp
(86) International application number: PCT/US2008/055904
(87) International publication number: WO 2008/109668

(56) References cited:
- WO-A-00/75275
- WO-A-95/04813
- WO-A-95/11048
- WO-A-03/105663
- US-A- 4 391 912
- US-A- 4 647 539
- US-A- 4 885 087
- US-A- 5 622 857
- US-A- 5 981 211
- US-A- 6 001 585

## Description

### BACKGROUND

Human stem cells, which have been expanded in culture from a small amount of donor cells, can be used to repair or replace damaged or defective tissues and have broad clinical applications for treatment of a wide range of diseases. Recent advances in the area of regenerative medicine demonstrate that stem cells have unique properties such as self-renewal capacity, the ability to maintain the unspecialized state, and the ability to differentiate into specialized cells under particular conditions.

As an important component of regenerative medicine, the bioreactor or cell expansion system plays a role in providing optimized environments for cell growth and expansion. The bioreactor provides nutrients to the cells and removal of metabolites, as well as furnishing a physiochemical environment conducive to cell growth in a closed, sterile system. Cell expansion systems can be used to grow other types of cells as well as stem cells.

US 6,001,585 discloses a bioreactor including an oxygen permeable tube containing a hollow fiber bundle.

WO 95/04813 discloses a hollow fiber bioreactor connected to an oxygenator.

US 4, 885, 087 discloses a bioreactor with valves to change the direction of flow.

Many types of bioreactors are currently available. Two of the most common include flat plate bioreactors and hollow fiber bioreactors. Flat plate bioreactors enable cells to grow on large flat surfaces, while hollow fiber bioreactors enable cells to grow either on the inside or outside of the hollow fibers.

If hollow fiber bioreactors are used, it is desirable to load cells into the hollow fibers in such a way that the cells are properly distributed throughout the length and width of the hollow fibers, not just at one end. It is to such aspects that the present invention is directed.

### BRIEF DESCRIPTION OF THE FIGURES

FIG. 1 is a schematic view of a bioreactor useful in this invention.
FIG. 2 is a flow diagram of a cell expansion system which may be used with the present invention.

### SUMMARY OF THE INVENTION

The invention is defined in the claims.

This invention is directed toward a method for moving cells in a hollow fiber bioreactor wherein the hollow fibers have an intracapillary space and an extracapillary space. The method includes the steps of loading cells into the intracapillary space, and flowing a fluid into one of the intracapillary space or extracapillary space at a flow rate pressure to move and distribute the cells along a length of the fibers.

This disclosure is also directed toward a method for reseeding cells contained in a hollow fiber bioreactor wherein the hollow fibers have an intracapillary space. This method includes the steps of flowing a fluid into the intracapillary space at a flow rate pressure to move the cells away from the walls of the hollow fibers; moving the cells out of the bioreactor; and returning the removed cells to the bioreactor to be reseeded.

### DETAILED DESCRIPTION

As discussed above, a number of bioreactor configurations exist for culturing cells, and it should be noted that this invention only requires that the bioreactor be equipped with an intracapillary and an extracapillary space.

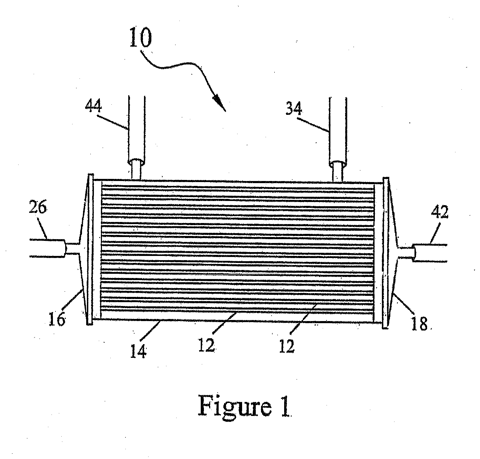

However, as but one example, not meant to be limiting, is a hollow fiber bioreactor shown in FIG. 1. A cell expansion module or bioreactor **10** which may be used in the present invention, is made of a bundle of hollow fiber membranes **12** enclosed within a housing **14.** The housing or module **14** may be cylindrical in shape and may be made of any type of biocompatible polymeric material. The hollow fibers are collectively referred to as a membrane. The space or lumen within the hollow fibers is defined as the intracapillary space (IC space), and the space surrounding the outside of the hollow fibers is defined as the extracapillary space (EC space). As described, the cells are grown and distributed in the IC space. Alternatively, the cells may be grown in the EC space and the same principles can apply.

Each end of the module **14** is closed off with end caps or headers **16**, **18**. The end caps **16, 18** may be made of any suitable material such as polycarbonate so long as the material is biocompatible with the cells to be grown in the bioreactor.

Approximately 9000 fibers **12** around 295 mm in length may be held in place within the housing **14** with polyethylene potting (not shown). The fibers **12** and potting may be cut through at each end to permit fluid flow into and out of the IC space. It is understood, however, the membrane and length of the fibers can be varied as this is only exemplary.

There may be at least four ports into and out of the module. Two ports fluidly connect to the extracapillary space, one port **34** for example, for extracapillary media egress into the space surrounding the hollow fibers and one port **44** for extracapillary media egress out of the module. Two ports also fluidly connect to the intracapillary space, one port **26** for intracapillary media egress into the lumens of the hollow fibers as well as egress of the cells to be expanded, and one port **42** for intracapillary media egress and for expanded cells to be recirculated or removed from the bioreactor. The ports shown may be called inlet or outlet or removal ports. It is understood that the fluids could also flow in directions opposite to those described.

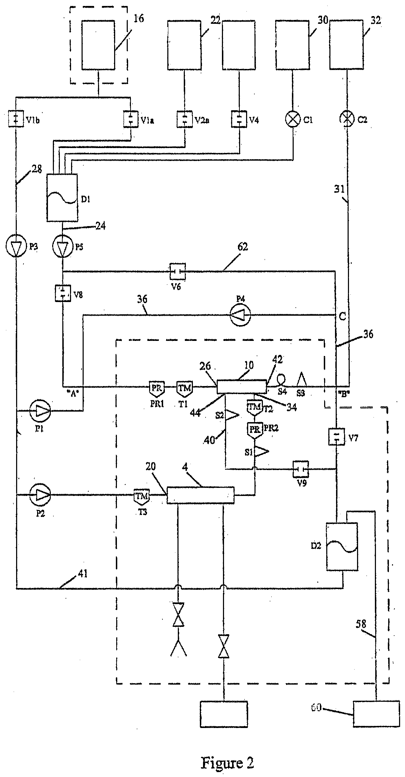

Cells to be expanded in the bioreactor may be flowed into the intracapillary or IC space of the fibers in the example described. The fibers may be loaded with cells using a syringe or the cells may be distributed into the intracapillary spaces directly from a container containing the cells. The cells may be added to the fibers in the fluid used for ultrafiltration or media as described below. The cells may also be introduced into the growth module or bioreactor from a cell input bag (**30,** see FIG. 2), which may be sterile docked directly to the IC space of the bioreactor.

The space between the fibers (EC space) may be used as a medium reservoir to supply nutrients to the cells and remove the byproducts of cellular metabolism. If cells are grown in the EC space, the IC space may be used as the medium reservoir to supply nutrients and remove the byproducts of cellular metabolism. This media may be replaced as needed. Media may also be circulated through an oxygenator **4** (see FIG. 2) to exchange gasses as needed. Growth media may also be provided in the hollow fiber space with the cells.

The hollow fibers may be made of a semi-permeable, biocompatible polymeric material. One such polymeric material which can be used is a blend of polyamide, polyarylethersulfone and polyvinylpyrrolidone. The semi-permeable membrane allows transfer of nutrients, waste and gases through the pores in the membrane between the EC and IC spaces. The molecular transfer characteristics of the hollow fiber membranes are chosen to minimize loss of expensive reagents necessary for cell growth such as growth factors, cytokines etc. from the IC side or the cell side, while allowing metabolic waste products to diffuse through the membrane into the EC or acellular side to be removed.

In a bioreactor, a semi-permeable membrane such as the material described above may be used to move molecules between the IC and EC compartments by either diffusion or convection.

Diffusion is accomplished by establishing a concentration gradient across the semi-permeable membrane. Molecules diffuse from the high concentration side to the low concentration side with a rate dependent upon the concentration difference and the membrane permeability.

Molecular convection occurs when a fluid flow is imposed across the membrane and is accompanied by a corresponding pressure drop across the membrane. This fluid flow or ultrafiltrate (UF) flow carries across the membrane, any waste products or media except for those products which are unable to cross the membrane due to pore size restrictions of the membrane or surface charges of either the products or the membrane.

Ultrafiltration or fluid flow across the membrane may also be used to aid in the movement and redistribution of cells within the fibers.

Differences in pressure caused by the flow of fluid between the membrane interior (IC side) and the membrane exterior (EC side) is termed transmembrane pressure (hereinafter referred to as TMP). If the pressure inside the hollow fiber exceeds the pressure in the area surrounding the fibers, this pressure differential tends to force the fluid outwardly through the membrane wall of the fiber. The semi-permeable hollow fiber membrane walls contain extremely small-diameter pores. These pores may be too small for larger components such as cells to pass through. Thus, the cells continue flowing through the interior of the hollow fiber. Water and other components--those small enough to pass through the pores in the membrane--are pushed in varying quantities through the membrane pores. The smaller the components, the easier they will flow through the fiber membrane and into the EC space, for a given pore diameter. Similarly, a greater transmembrane pressure causes a higher rate of filtration, i.e., a higher rate of UF flow into the EC space. The more the hollow fiber interior fluid flow pressure exceeds the exterior chamber fluid flow pressure, the greater the force exerted to push components through the membrane and into the EC space.

Speeding up the pump and increasing flow or the flow rate through the hollow fibers will raise the fiber interior pressure and, hence, the transmembrane pressure.

These principles of flow may be utilized to distribute cells throughout the hollow fibers, or to help push cells off the surfaces of the hollow fibers in order to reseed or harvest the expanded cells.

For the purposes of the explanation below positive flow is described as higher pressure on the IC side causing flow to the EC side. Negative flow is described as higher pressure on the EC side causing flow to the IC side.

As but one example, not meant to be limiting, in a cell expansion system which includes the hollow fiber bioreactor described above, fluid flow can be regulated using various pumps and/or valves which may be part of the system. A schematic of a possible cell expansion system is shown in FIG. 2. Other cell expansion systems, which may also be used with this invention, are disclosed in patent application PCT/US08/55915 (WO2008/109674) filed March 5, 2008. Only the portions of FIG. 2 necessary to accomplish the purposes of this invention will be discussed.

An EC media bag **16** contains EC media so that such media will flow through the EC side of the bioreactor **10** and may be connected via a portion of flexible tubing (the EC inlet line) **28** to the EC inlet port **20** of an oxygenator **4.** The EC inlet line **28** brings fresh EC media to the oxygenator **4** to be oxygenated. From the oxygenator the EC media flows to EC inlet **34** through the bioreactor to outlet **44.**

An IC media bag **22,** containing the IC media so that such media will flow through the IC side of the bioreactor, may be connected via a portion of flexible tubing (the IC inlet line) **24** to the IC inlet port **26** of the bioreactor **10.** The IC inlet line **24** brings fresh IC media to the IC side of the bioreactor.

A cell input bag **30** contains the cells to be expanded in the bioreactor **10.** The cell input bag **30** is connected to the IC inlet line **24** which delivers cells into the lumen of the hollow fibers via IC inlet port **26.**

When the cells are ready to be harvested, they are flushed out of the IC outlet port **42** of bioreactor **10** through cell harvest line **31** and into a cell harvest bag **32.**

Waste from the EC side may be flushed through valve **V9** and line **58** to waste bag **60.**

The cell growth system also may include a length of tubing which acts as an IC circulation loop **36.** The IC media flows out of the bioreactor **10** from the IC outlet port **42** through tubing loop **36** and back into the bioreactor through the IC inlet port **26.** This loop **36** is used to recirculate the IC media though the hollow fibers. It may also be used to flush the cells out of the hollow fibers and reseed/redistribute them throughout the hollow fibers for further expansion as more fully described below.

Also an EC recirculation loop including lines **40** and **41** and pump **P2** may be provided to recirculate on the EC side.

Additional tubing line **62** can be added as needed to enable specific applications such as reseeding/redistributing cells in the bioreactor.

As described below, the bioreactor system can utilize multiple pumps to increase the flexibility of the system.

With the cells to be expanded on the IC side, the nutrient media circulates through the EC side of the bioreactor. The cells will consume certain nutrient components from the EC fluid and release metabolic waste products back into the EC media. It is important that the nutrient fluid be replaced to assure satisfactory cell culture. This is accomplished by at least one pump **P3.**

**P3** pumps fresh replacement media from the replacement media bag **16** (EC media bag) into the EC side of the bioreactor. In one embodiment, **P3** pumps around 500 mL of replacement media into the system at a speed of around 50 mL/min. The frequency of media replacement is dependent upon several factors such as the number of cells in the bioreactor and the amount of metabolic waste products produced by the cells, however the average media replacement may be around every two days.

**P3** may be user definable, that is, the user can control the flow of replacement media if certain conditions are desired. For example, if it is desired to clean or flush out the system, a higher flow rate and higher amount of media may be chosen by the user. The EC media replacement or supplementation to the media already in the bioreactor may also occur on a slow continuous basis. For example around 0.2 mL/min of fresh media may be continuously released into the system. The speed of **P3** may also be increased to generate a negative flow on the EC side of the bioreactor so that IC fluid will cross the membrane from the EC side to the IC side.

Pump **P5** may be used to pump fresh IC media from IC media bag **22** and cells from cell input bag **30** into the hollow fibers (IC space) of the bioreactor. This pump may also be used for priming the IC space of the bioreactor with IC media to flush out any air which may be present in the fibers before the cells to be expanded are seeded within the fibers. This pump may also be used if the IC media needs to be replaced, or if fresh IC media containing a different proportion of cytokines or growth factors is desired. The speed of **P5** may be increased to generate a positive flow on the IC side of the bioreactor as compared to the EC fluid flow as described below.

In an alternative embodiment, an additional pump could be added to the basic system to re-circulate IC media and cells. As described below, pump **P4** may be used as an intracapillary pump for recirculating IC media and/or through the bioreactor. **P4** also can be used to create a shear flow rate over the cells within the IC space, which may help to lift the cells from the fiber surface and reseed or redistribute them within the IC space. **P4** may also be used to remove cells from the bioreactor either to reseed them back into the bioreactor for further expansion or to collect them in a cell harvest bag. **P4** may be increased to create positive flow or ultrafiltrate flow from the IC side to the EC side across the membrane.

The operational speed of pumps **P1-P4** and the diameter of the tubes are selected so as to produce a flow rate through the tubes of between 0-150 mL per minute during operation of the pumps. **P5** can produce a flow rate of between 0-250 mL/min.

### Example 1

Tubing lines **36** and **62** can be used to redistribute and/or recirculate both adherent and suspension cells on the IC side.

With both adherent and suspension cultures growing in a bioreactor (specifically a hollow fiber bioreactor) there are occasions when it is desirable to redistribute the growing cells throughout the fibers in the bioreactor. If non-adherent cells are being grown, it may also be desirable to continuously recirculate the cells throughout the tubing and bioreactor. If adherent cells are being grown they must first be released from the membrane using common techniques such as shear rate, negative flow (described below), change in calcium concentration, trypsin and/or other chemical or physical methods including cold or heat.

In this procedure, a recirculation line (see for example tubing loop **36** in FIG. 2) is provided. This path allows the cells to leave the bioreactor **10** via outlet port **42** and under the pumping action of **P4** the cells then re-enter IC inlet line **24** at a position **"A"** near the inlet port **26.** There may also be a source of media **22** for back flushing the cells. From the point of fresh media entry, the cells may be flushed in both directions as described below to ensure the cells are flushed from the recirculation line **36** back into the bioreactor.

With valves **v6** and **v8** closed, pump **P4** can be used to circulate the cells in the resulting closed loop **36** through the bioreactor **10** until the desired uniformity of cell mixing is achieved.

After the cells are effectively mixed, valve **v6** is opened and valve **v8** is closed, pump **P5** is activated and pump **P4** is set to pump at a lower speed than pump **P5.** Pump **P5** will pump IC media into the system. Pump **P4** will effectively divert a portion of the recirculated flow towards the bioreactor inlet **26** with the remainder forced to the bioreactor outlet **42** by the flow of media through lines **62** and **36** and valve **V6.** Both pumps will effectively flush cells back into the bioreactor. Any excess fluid going into the bioreactor will be forced through the hollow fibers as ultrafiltrate if the IC fluid flow pressure is greater than the EC fluid flow pressure. The ultrafiltrate will also push the cells toward the hollow fiber walls. The position at which the back flush line **62** connects to the recirculation line **36** (shown in FIG. 2 as junction **C)** and the volume/number of cells on either side of this connection will determine how many cells are redistributed back through the outlet **42.** This point of connection could be placed so close to the bioreactor that effectively all the cells would be redistributed by way of the bioreactor inlet port **26,** possibly even eliminating the need to flush media in both directions.

The above shows how cells can be distributed and recirculated through the bioreactor using ultrafitration and back flushing. The process could be the same for adherent cells after such cells are released from the membrane by adding a chemical release agent or other methods as described above.

Alternatively, the cells could be reseeded in the bioreactor by first being removed from the bioreactor and tubing into cell harvest bag **32.** To reseed the cells, the harvest bag **32** may be attached to bioreactor inlet port **26,** in the same manner as for the loading of the cells.

In another alternative, cells could be removed from the bioreactor into cell harvest bag **32.** Any cells remaining in the bioreactor could then be expanded. This process could be repeated in a continuous manner.

Further describing FIG. 2, an EC recirculation loop **40** allows the media on the EC side of the bioreactor to be recirculated. The EC recirculation loop **40** allows EC media to flow out of the bioreactor from the EC outlet port **44** back into the bioreactor through the EC inlet port **34.** This loop may be used to recirculate the EC media which surrounds the hollow fibers, bringing nutrients from one portion of the bioreactor to another. By keeping the EC fluid flow less than the IC fluid flow, the IC fluid flow pressure will be greater than the EC fluid flow pressure, allowing the IC fluid flow to flow across the membrane, creating a positive fluid flow.

Alternatively if it is desirable to grow the cells on the EC side, the EC fluid flow can be greater than the fluid flow on the IC side, to force fluid from the EC side to the IC side, creating a negative fluid flow. This flow would be under the force of pumps **P3** and **P2.** If **P3** is greater than **P2,** back flushing in outlet **44** can occur to help in reseeding the EC side.

IC and EC media containing metabolic breakdown products from cell growth are removed from the system via tubing **58** into a waste bag **60.**

### Example 2

Using positive ultrafiltration to assist in the attachment of substantially purified MSCs to the membrane.

If adherent cells such as mesenchymal stem cells (MSCs) are to be expanded in a hollow fiber bioreactor, the cells must first attach to the surface of the fibers to begin their normal growth cycle. To enhance/promote this attachment, a positive pressure caused by increased fluid flow on the IC side could be applied, i.e. the pressure in the cell side compartment (IC side) being higher than the pressure in the non-cell side (EC side). In the cell expansion system described above, positive flow could be achieved by increasing the pump speed and therefore the flow of fluid or fluid pressure on the IC side. This is done by increasing the speed of pump **P5** which controls the flow of IC media into the hollow fibers of the bioreactor.

As discussed above, the increased flow of fluid will cause a flow of fluid through the fibers, creating a positive flow, which will assist in dragging the MSCs to the fiber wall. Such flow is advantageous because this flow will drag cells to all parts of the cell fiber surface, where if only gravity was used to distribute the cells in the bioreactor, the cells would predominately settle in the bioreactor header **16** (see FIG. 1) or only a short distance into the fibers, not along the entire length and circumference of the fibers.

A positive ultrafiltration rate or positive flow could continue to be applied (possibly at a reduced level) to hold the MSCs at the fiber surface until the cells attach, possibly for a few hours to a few days.

This method is most useful where the cells to be expanded are substantially purified before they are added to the bioreactor. For the purposes of this example, substantially purified means one cell type is predominant as compared to other cell types in a cell suspension.

After the attachment period, a substantially zero fluid flow or slightly negative fluid flow across the fibers could be used to cause any cells which did not attach to the membrane to be removed from the bioreactor. The negative fluid flow can be produced by dropping the IC flow rate pressure as compared to that of the EC side.

If non-adherent cells were grown in a hollow fiber bioreactor, positive flow could also be used to hold the cells against the fiber walls to prevent the cells from being pushed out of the bioreactor when the old IC media was replaced with fresh IC media.

### Example 3

Using negative flow or reverse ultrafiltration to create cell suspend mode.

A negative fluid flow or reverse ultrafiltration could be used to assist in keeping cells in a suspension mode. For example, negative flow produces a flow of fluid from the EC side to the IC side. The flow of fluid into the fibers will push the cells away from the wall. In the cell expansion system described above, negative flow may be achieved by increasing the speed of pump **P2** which controls the flow of EC fluid into the bioreactor, closing valve **v9,** and opening clamp **c2.** In addition to or alternatively, valve **v7** could be opened, and the speed of pump **P3** increased so that the speed of **P3** is greater than or equal to the speed of pump **P2.**

Combinations of positive and negative flow could be used for loading cells into the bioreactor. Negative flow could be used to create a suspension mode in the entrance of the bioreactor followed by a region in the bioreactor of positive flow where cell suspension was no longer maintained (i.e. an adhesion mode). By utilizing such combinations of positive and negative fluid flow one could prevent the deposition of cells in a first region of the bioreactor and enhance the deposit of cells in a second region.

### Example 4

Use of positive and negative fluid flow to distribute and remove non-adherent cells from a hollow fiber bioreactor.

As discussed in Example 2, cells to be expanded in a hollow fiber bioreactor can be purified first, before being loaded into the bioreactor. However, unpurified cells such as whole bone marrow can also be loaded directly into a hollow fiber bioreactor. Positive flow can be used first to help the adherent cell portion of the whole bone marrow adhere to the fibers, followed by application of negative flow to help flush the non-adherent cell portion of the whole bone marrow such as red blood cells, white blood cells and platelets out of the hollow fibers.

50 mL whole bone marrow (which is the amount typically drawn directly from a single bone marrow draw) may be flowed directly from cell input bag **30** (see FIG. 2) through the IC inlet port **26** into the hollow fibers. The IC outlet port **42** is clamped during loading to prevent cell loss as well as to create a positive fluid flow. Once loaded, the bone marrow is incubated for between around 1-4 days to allow the adherent cells in the bone marrow time to adhere to the fibers. Alternatively, positive ultrafiltration or fluid flow can be applied to help drag the cells to the membrane to help the cells adhere.

Negative flow, after the opening of outlet **42,** can then be applied to push all superfluous cells, which are not adhered to the membrane, out of the bioreactor. This is done by using **P3** to increase the flow rate of EC media through the bioreactor. **P2** and **P3** create negative flow in the bioreactor to lift the non-adherent cells off the fibers and flush them out of the fibers, leaving only those cells which have adhered to the fibers in the bioreactor.

### Example 5

Selective distribution in hollow fibers using density gradients.

The IC space and EC spaces of the bioreactor could be initially filled with media having a density d₁. The cells to be grown in the bioreactor may be suspended in a media having density d₂ where d₂ > d₁ and then loaded into the IC space of the bioreactor. The bioreactor could be held horizontally, and if one used a slow cell inlet flow rate the cells (in heavier media) would fall to the bottom of the bioreactor inlet header and then flow into only those fibers at the bottom of the bioreactor. The use of viscosity (µ₁, µ₂) and flow rate could also be helpful in positioning the cells within the fibers.

### Example 6

Using fluid flow to distribute mitotic cells along a hollow fiber.

During cell division or mitosis, adherent cells tend to pull away from the membrane on which they are adhered, or at least become more loosely attached thereto.

In this embodiment, cells are grown in the IC side of the membrane and a flow of media is imposed through the hollow fibers. This flow of fluid through the hollow fibers imparts a shear force on the cells. This force is used to help lift cells undergoing mitosis off of the membrane and move the lifted cells down the hollow fiber with the media stream.

The fluid flow through the membrane must be slow enough to allow the lifted cells to fall out of the media stream. Once out of the media stream, the cells will re-attach to the membrane at more downstream locations, thus facilitating re-distribution and growth at more locations along the fiber.

Selecting combinations of pumps that have varying flow outputs could also be used to regulate fluid flow through the fibers. Such pump combinations could be used to create periods of higher shear to release cells from the membrane, followed by periods of lower shear to allow cells to settle and re-attach to the membrane.

Such a method could also be used to initially load cells to be expanded into the bioreactor at the inlet to the hollow fibers and using the method of intermittent flow to move the cells down the fibers.

The examples given above are several of the applications which could be utilized following the principles of the present invention and are not meant to limit the scope of the present invention as defined by the attached claims.

## Claims

1. A method for moving cells in a hollow fiber bioreactor (10) wherein the hollow fibers (12) have an intracapillary space and an extracapillary space, the method comprises the steps of:
loading cells into the intracapillary space or extracapillary space;
flowing fluid into one of the intracapillary space or extracapillary space containing the cells; and
providing a fluid flow pressure in one of the intracapillary space or extracapillary space greater than the fluid flow pressure in the other of the intracapillary or extracapillary space.

2. The method of claim 1 wherein the step of providing a fluid flow pressure comprises changing the fluid flow pressure in the space containing the cells as compared with the other of the intracapillary or extracapillary space.

3. The method of claim 2 further comprising increasing the fluid flow pressure by increasing the fluid volume in the space containing the cells.

4. The method of claim 1 wherein the cells to be moved are in the intracapillary space.

5. The method of claim 1 further comprising moving the cells to the surface of the hollow fibers.

6. The method of claim 1 wherein the hollow fibers comprise pores.

7. The method of claim 6 wherein the step of flowing fluid further comprises flowing part of the fluid from the space containing the cells to the other of the intracapillary space or extracapillary space through the pores of the hollow fiber.

8. The method of claim 5 wherein the step of moving the cells to the surface of the hollow fibers further comprises allowing the cells to adhere to the surface of the hollow fibers.

9. The method of claim 3 wherein increasing the fluid flow pressure comprises increasing fluid pump speed to increase the fluid flow rate.

10. The method of claim 5 wherein the step of flowing fluid comprises holding the cells on the surface of the hollow fibers.

11. The method of claim 1 further comprising maintaining the cells in a suspension mode within the space.

12. The method of claim 1 further comprising moving the cells away from the surface of the hollow fibers.

13. The method of claim 12 wherein the step of moving the cells away from the walls of the hollow fibers further comprises releasing cells from the walls of the hollow fibers.

14. The method of claim 13 wherein the step of releasing cells from the walls of the hollow fibers further comprises adding a release chemical to the fluid.

## Patentansprüche

1. Verfahren zum Bewegen von Zellen in einen Hohlfaserbioreaktor (10), wobei die Hohlfasern (12) einen intrakapillaren Raum und einen extrakapillaren Raum haben, wobei das Verfahren die folgenden Schritte umfasst:
Laden von Zellen in den intrakapillaren Raum oder extrakapillaren Raum;
Strömen von Fluid in einen von dem intrakapillaren Raum oder extrakapillaren Raum, der die Zellen enthält; und
Bereitstellen eines Fluidströmungsdrucks in einem von dem intrakapillaren Raum oder extrakapillaren Raum, der größer als der Fluidströmungsdruck in dem anderen von dem intrakapillaren oder extrakapillaren Raum ist.

2. Verfahren nach Anspruch 1, wobei der Schritt eines Bereitstellens eines Fluidströmungsdrucks ein Verändern des Fluidströmungsdrucks in den Raum, der die Zellen enthält, verglichen mit dem anderen von dem intrakapillaren oder extrakapillaren Raum umfasst.

3. Verfahren nach Anspruch 2, ferner umfassend ein Erhöhen des Fluidströmungsdrucks, indem das Fluidvolumen in dem Raum, der die Zellen enthält, erhöht wird.

4. Verfahren nach Anspruch 1, wobei die zu bewegenden Zellen in dem intrakapillaren Raum sind.

5. Verfahren nach Anspruch 1, ferner umfassend ein Bewegen der Zellen zu der Oberfläche der Hohlfasern.

6. Verfahren nach Anspruch 1, wobei die Hohlfasern Poren umfassen.

7. Verfahren nach Anspruch 6, wobei der Schritt eines Strömens von Fluid ferner ein Strömen eines Teils des Fluids von dem Raum, der die Zellen enthält, zu dem anderen von dem intrakapillaren Raum oder extrakapillaren Raum durch die Poren der Hohlfaser umfasst.

8. Verfahren nach Anspruch 5, wobei der Schritt eines Bewegens der Zellen zu der Oberfläche der Hohlfasern ferner ein Zulassen umfasst, dass die Zellen an der Oberfläche der Hohlfasen anhaften.

9. Verfahren nach Anspruch 3, wobei ein Erhöhen des Fluidströmungsdrucks ein Erhöhen der Fluidpumpgeschwindigkeit umfasst, um die Fluidströmungsrate zu erhöhen.

10. Verfahren nach Anspruch 5, wobei der Schritt eines Strömens von Fluid ein Halten der Zellen an der Oberfläche der Hohlfasern umfasst.

11. Verfahren nach Anspruch 1, ferner umfassend ein Beibehalten der Zellen in einem Suspensionsmodus in dem Raum.

12. Verfahren nach Anspruch 1, ferner umfassend ein Bewegen der Zellen weg von der Oberfläche der Hohlfasern.

13. Verfahren nach Anspruch 12, wobei der Schritt eines Bewegens der Zellen weg von den Wänden der Hohlfasern ferner ein Freisetzen von Zellen von den Wänden der Hohlfasern umfasst.

14. Verfahren nach Anspruch 13, wobei der Schritt eines Freisetzens von Zellen von den Wänden der Hohlfasern ferner ein Hinzufügen einer Freisetzungschemikalie zu dem Fluid umfasst.

## Revendications

1. Procédé pour déplacer des cellules dans un bioréacteur à fibres creuses (10) dans lequel les fibres creuses (12) possèdent un espace intra-capillaire et un espace extra-capillaire, le procédé comprend les étapes de :
le chargement de cellules dans l'espace intra-capillaire ou l'espace extra-capillaire ;
l'écoulement de fluide dans l'un de l'espace intra-capillaire ou de l'espace extra-capillaire contenant les cellules ; et
la fourniture d'une pression d'écoulement de fluide dans l'un de l'espace intra-capillaire ou de l'espace extra-capillaire plus élevée que la pression d'écoulement de fluide dans l'autre de l'espace intra-capillaire ou de l'espace extra-capillaire.

2. Procédé selon la revendication 1, dans lequel l'étape de la fourniture d'une pression d'écoulement de fluide comprend le changement de la pression d'écoulement de fluide dans l'espace contenant les cellules par rapport à l'autre de l'espace intra-capillaire ou de l'espace extra-capillaire.

3. Procédé selon la revendication 2, comprenant en outre l'augmentation de la pression d'écoulement de fluide en augmentant le volume de fluide dans l'espace contenant les cellules.

4. Procédé selon la revendication 1, dans lequel les cellules destinées à être déplacées sont dans l'espace intra-capillaire.

5. Procédé selon la revendication 1, comprenant en outre le déplacement des cellules vers la surface des fibres creuses.

6. Procédé selon la revendication 1, dans lequel les fibres creuses comprennent des pores.

7. Procédé selon la revendication 6, dans lequel l'étape de l'écoulement de fluide comprend en outre l'écoulement d'une partie du fluide, de l'espace contenant les cellules à l'autre de l'espace intra-capillaire ou de l'espace extra-capillaire, à travers les pores de la fibre creuse.

8. Procédé selon la revendication 5, dans lequel l'étape du déplacement des cellules vers la surface des fibres creuses comprend en outre la permission, aux cellules, d'adhérer à la surface des fibres creuses.

9. Procédé selon la revendication 3, dans lequel l'augmentation de la pression d'écoulement de fluide comprend l'augmentation de vitesse de pompe à fluide pour augmenter le débit de fluide.

10. Procédé selon la revendication 5, dans lequel l'étape de l'écoulement de fluide comprend le maintien des cellules sur la surface des fibres creuses.

11. Procédé selon la revendication 1, comprenant en outre le maintien des cellules dans un mode en suspension à l'intérieur de l'espace.

12. Procédé selon la revendication 1, comprenant en outre le déplacement des cellules pour les éloigner de la surface des fibres creuses.

13. Procédé selon la revendication 12, dans lequel l'étape du déplacement des cellules pour les éloigner des parois des fibres creuses comprend en outre la libération de cellules à partir des parois des fibres creuses.

14. Procédé selon la revendication 13, dans lequel l'étape de la libération de cellules à partir des parois des fibres creuses comprend en outre l'ajout, au fluide, d'un produit chimique de libération.
